# EUROPÄISCHE PATENTANMELDUNG

(11) **EP 1 600 105 A2**
(43) Veröffentlichungstag der Anmeldung: **30.11.2005**
(21) Anmeldenummer: 05011433.9
(22) Anmeldetag: 27.05.2005
(51) Int. Cl.: A61B 7/02

(54) **Doppelstethoskop**

(30) Priorität: 29.05.2004 DE 202004008577 U
(71) Anmelder: Barthelmes, Arno, 98544 Zella-Mehlis (DE)
(72) Erfinder: Barthelmes, Arno, 98544 Zella-Mehlis (DE)
(74) Vertreter: Engel, Christoph Klaus

(57) **Zusammenfassung**

Die Erfindung betrifft ein Doppelstethoskop mit einem Schalltrichter (7), der einen Schallkollektorraum (8) umfasst und der über einen Schlauchanschlusskörper (1) zum Übertragen von Geräuschen an ein Schlauchsystem (5) angeschlossen ist. Erfindungsgemäß besteht der Schalltrichter (7) aus einem weichelastischen Material und ist über ein hohles Anschlussstück (6) mit dem Schlauchanschlusskörper (1) verbunden. An dem zum Schallkollektorraum (8) gerichteten Ende des hohlen Anschlussstücks (6) ist eine erste umlaufende Abdichtwulst (9) angeordnet, während an der Öffnung des Schalltrichters (7) eine zweite umlaufende Abdichtwulst (9a) angeordnet ist. Vorzugsweise ist dem weichelastischen Material des Schalltrichters (7) Nanosilber zugesetzt ist.

## Beschreibung

Die Erfindung betrifft ein Doppelstethoskop mit einem Schalltrichter, der einen Schallkollektorraum umfasst und der über einen Schlauchanschlusskörper zum Übertragen von Geräuschen an ein Schlauchsystem angeschlossen ist. Vorzugsweise ist das Schlauchsystem mit zwei Ohrstücken ausgerüstet, zum Übertragen von Geräuschen in die Ohrmuscheln des Benutzers.

Stethoskope gibt es in den verschiedensten Ausführungen als Einfach- und Doppelstethoskope. Einfachstethoskope sind mit einem Membranstück oder mit einem offenen Trichter versehen, somit einfach und leicht im Aufbau sowie preiswert in der Anschaffung. Ihre Einsatzmöglichkeit gegenüber dem Doppelstethoskop ist jedoch begrenzt. Doppelstethoskope besitzen ein zweiseitig verwendbares Bruststück, wobei die eine Seite in der Regel mit einer Membrane ausgestattet ist und die gegenüberliegende mit einem offenen Trichter.

So beschreibt die DE-OS 26 07 990 ein doppelköpfiges Stethoskop, das geringe Abmessungen aufweist und die wechselweise Benutzung der Schalltrichter ermöglichen soll. Dieses Stethoskop besteht aus zwei Schalltrichtern unterschiedlicher Größe. Sie sind durch Drehen aus Metall oder Kunststoff geformt, wobei zwischen beiden Schalltrichtern durch Ausdrehen ein zylindrisches Übergangsstück gebildet wird. Die Schallkollektorräume der beiden Schalltrichter sind hierbei durch eine Bohrung verbunden, so dass beide Schalltrichter eine gemeinsame Schallleitungsbohrung aufweisen, in welcher ein Rohrstutzen mündet, an welchem in bekannter Weise der Schallleitungsschlauch angeschlossen ist.

Der Schallkollektorraum des großen Schalltrichters ist mit einer Membrane überspannt, die am Umfang des Schalltrichters befestigt ist. Der kleine Schalltrichter dagegen ist nicht mit einer Membrane versehen. Auf dem Rohrstutzen ist ein Rohrstück angebracht, das mit einer Rändelung versehen ist, so dass durch ein schnelles Umdrehen ein schnelles wechselweises Ansetzen der Schalltrichter an das auszukultierende Objekt möglich sein soll.

Wird beim Auskultieren der größere Schalltrichter verwendet, wird die Mündung der Schalltrichterbohrung im kleineren Schalltrichter mit einer Fingerbeere verschlossen. Wird dagegen der kleinere Schalltrichter angesetzt, so wird die Membrane des großen Schalltrichters mit einem Finger so weit eingedrückt, dass sie die Mündung der Schallleitungsbohrung verschließt. Somit sollen Nebengeräusche beim Auskultieren vermieden werden.

Die beschriebene Art der Stethoskope besitzt den Nachteil, dass eine mechanische Abnutzung durch das Umdrehen bei wechselweisem Ansetzen der Schalltrichter nach einer längerfristigen Benutzung des Stethoskops eintritt. Darüber hinaus besitzen diese Stethoskope einen festen Aufbau, welcher in bestimmten Zeitabschnitten zum Zwecke der Wartung und Reinigung demontiert werden muss.

Um diese Nachteile auszuschließen, beschreibt das DE-GM 298 18 777 ein Stethoskop, welches es dem Arzt ermöglichen soll, rasch zwischen unterschiedlichen Bruststücken, die ebenfalls gegenüberliegende Schalltrichter aufweisen, zu wechseln, während gleichzeitig Wartung und Reinigung vereinfacht werden können. Des Weiteren soll dem Benutzer gestattet werden, nur einen Teil des Stethoskops auszutauschen, wenn dieser Teil schadhaft ist, um damit Kosten der Reparatur oder für einen Neukauf zu reduzieren.

Das in der DE-GM 298 18 777 beschriebene Stethoskop umfasst ein Schlauchsystem, an welches ein Adapter lösbar montiert ist. Eine Vielzahl von Bruststücken ist hierbei selektiv und austauschbar mit dem Adapter verbindbar. Das Bruststück besitzt einen rohrförmigen Bereich mit einem Führungsschlitz, der sich in axialer Richtung erstreckt sowie einem Arretierungsschlitz, welcher im Umfang verläuft und in Verbindung mit dem Führungsschlitz steht. Der rohrförmige Bereich des Bruststücks trägt eine tiefe Ausnehmung, die an dem Halteschlitz ausgebildet ist. Der Adapter besitzt eine Bohrung zur Aufnahme des rohrförmigen Bereiches des Bruststückes. Die Bohrung besitzt eine sich radial erstreckende Ausnehmung, innerhalb welcher ein kugelförmiges Element verschiebbar behalten ist und unter dem Druck einer Feder steht, wobei es teilweise in die Bohrung hineinragt, so dass dann, wenn der rohrförmige Teil des Bruststückes in die Bohrung hineingestreckt wird, das kugelförmige Element in dem Führungsschlitz und dem Arretierungsschlitz positioniert und geführt wird, so dass es die tiefe Ausnehmung erleichtert und darin fest gehalten wird, so dass eine lösbare Befestigung des Bruststückes an dem Adapter gegeben ist. Um das Bruststück aus dem Adapter herauszunehmen, ist es lediglich erforderlich, unter Kraftaufwand das Bruststück in bezug auf den Adapter zu drehen, um das kugelförmige Element zurück in die sich radial erstreckende Bohrung hineinzuführen, worauf dann das Bruststück relativ zu dem Adapter entlang des Arretierungsschlitzes und dann entlang des Führungsschlitzes bewegt werden kann, um das Bruststück von dem Adapter zu trennen. Ein anderes Bruststück kann dann mit dem Adapter verbunden werden, um es dem Benutzer zu gestatten, zwischen den unterschiedlichen Bruststücken zu wechseln.

Die im Stand der Technik beschriebenen Doppelstethoskope besitzen den Nachteil, dass die beiden gegenüberliegenden Schalltrichter aus einem harten Werkstoff gefertigt sind. Dadurch besteht die Gefahr, dass beim Auskultieren die entsprechende Körperoberfläche unzureichend abgedeckt wird und somit auch äußere Fremdgeräusche durch den Untersuchenden mit wahrgenommen werden.

Das beschriebene Verschließen des eingesetzten Schalltrichters ist ebenfalls so aufwendig wie ein ständiges Wechseln der unterschiedlich großen Bruststücke. Auch ist es nicht nachvollziehbar, dass ein ständiges Auswechseln der Bruststücke die mechanische Abnutzung gegenüber dem Umdrehen des Bruststückes beim wechselweisen Ansetzen der Schalltrichter verringern soll.

Da die beschriebenen Doppelstethoskope aus einer Vielzahl von feinmechanischen Einzelteilen bestehen, ist deren Fertigung nicht nur zeit- und kostenintensiv, sondern auch gegen pathogene, multiresistente Hospitalkeime nur mit sehr hohem Aufwand antimikrobiell zu reinigen.

Der vorliegenden Erfindung liegt die Aufgabe zugrunde, ein Doppelstethoskop zu schaffen, das sowohl bei der allgemeinen Auskultation als auch beim interkostalen Aufsetzen die entsprechende Körperoberfläche ohne Verstellung einer Mechanik abdeckt und somit eine exakte Lokalisation zum Patienten ermöglicht. Eine Teilaufgabe der Erfindung besteht darin, ein Stethoskop bereitzustellen, das einen leicht zu desinfizierenden, kompakten Aufbau besitzt, leicht zu handhaben ist und das bei hohem Gebrauchswert und gutem Design zeit- und kostengünstig in der Herstellung ist.

Diese Aufgabe wird durch das in dem beigefügten Anspruch 1 näher definierte Doppelstethoskop gelöst.

Das erfindungsgemäße Doppelstethoskop besitzt zwei Schalltrichter, die einen Schallkollektorraum bilden. In an sich herkömmlicher Weise ist ein Schlauchanschlusskörper mit dem Schallkollektorraum verbunden, wobei ein Rohrstutzen in den Schlauchanschlusskörper hineinragt und ein Schlauchsystem mit zwei Ohrstücken an den Rohrstutzen angeschlossen ist, zum Übertragen von Geräuschen in die Ohrmuscheln des Benutzers.

Vorzugsweise ist der Schlauchanschlusskörper halbkugelförmig gestaltet und besitzt einen rotationsellipsoiden Schallraum, der über eine Schallleitungsbohrung und den Rohrstutzen akustisch mit dem Schlauchsystem verbunden ist. Die beiden Schalltrichter sind integral aus einem weichelastischen Kunststoff gefertigt, und besitzen einen vorzugsweise hohlzylindrisch gestalteten Anschlusskörper, der auf das Schlauchanschlussstück kraft- und form- oder stoffschlüssig aufgesetzt ist. Zur Ausbildung der beiden Schalltrichter ist vorzugsweise unterhalb des hohlzylindrischen Anschlussstückes in den Schallkollektorraum hineinragend eine erste umlaufende Abdichtwulst angeordnet, während an der Öffnung des Schalltrichters eine zweite umlaufende Abdichtwulst verläuft.

Mit der Erfindung wird erreicht, dass die von dem Schalltrichter aufgenommenen Körpergeräusche über den rotationsellipsoiden Schallraum des Schlauchanschlusskörpers ohne Verluste bis in das Ohr des Untersuchenden übertragen werden. Der weichelastische Kunststoff des Schalltrichters gewährleistet hierbei ein vollständiges Abdecken der abzuhörenden Körperstelle, so dass keine äußeren Fremdgeräusche mit aufgenommen werden. Das wechselseitige Benutzen der verschieden großen Schalltrichter erfolgt hierbei über eine unterschiedliche Druckausübung des Untersuchenden auf den Kopfteil.

Durch den kompakten zweiteiligen Aufbau ist der Kopfteil leicht zu reinigen und zu desinfizieren. In bezug auf die gewünschte einfache Reinigungsmöglichkeit ist eine Ausführungsform besonders vorteilhaft, bei welcher dem weichelastischen Kunststoff des Schalltrichters eine bestimmte Menge Nanosilber, vorzugsweise 2 Vol%, beigemengt ist. Damit besitzt das am Patienten angesetzte Kontaktteil des Stethoskops eine antimikrobielle, insbesondere antibakterielle Wirkung, da es selbstdesinfizierende Eigenschaften aufweist.

Vorteilhafte Ausgestaltungen der Erfindung sind in den Unteransprüchen angegeben.

Eine bevorzugte Weiterbildung, bei welcher die beiden umlaufenden Abdichtwulste im Querschnitt einen Radius aufweisen, gewährleistet einen dichten Abschluss des jeweils aktivierten Schalltrichters auf der Hautoberfläche des Patienten.

Um ein seitliches Abknicken des Schalltrichters während der Untersuchung zu vermeiden und den Schlauchanschlusskörper gegenüber dem Schalltrichter mittig zu halten, sind verschiedene Gestaltungen des Schalltrichters zwischen den beiden Abdichtwulsten möglich. Der Schlauchanschlusskörper wird vorzugsweise aus einem leichten, stabilen und gut zu reinigendem Material gefertigt, beispielsweise Edelstahl.

Als Material für die Ausbildung des Schalltrichters eignet sich vorzugsweise Polyurethan oder Silikon, da es bei diesen Materialien auch einfach möglich ist, das antimikrobiell wirkende Nanosilber in der gewünschten Homogenität einzubringen. Gleichzeitig lassen sich diese Materialien einfach und preiswert verarbeiten.

Der Kopfteil, bestehend aus einem Drehteil und einem Kunststoffgießteil, ist zeit- und kostengünstig in der Einzelfertigung und sehr leicht zu montieren.

Ein Ausführungsbeispiel der Erfindung wird anhand einer Figur erläutert. Sie zeigt eine Schnittdarstellung des Bruststückes des Doppelstethoskops mit einem kegelförmig ausgebildeten Schalltrichter, an dessen Außenfläche umlaufende Stabilisierungswulste angeordnet sind.

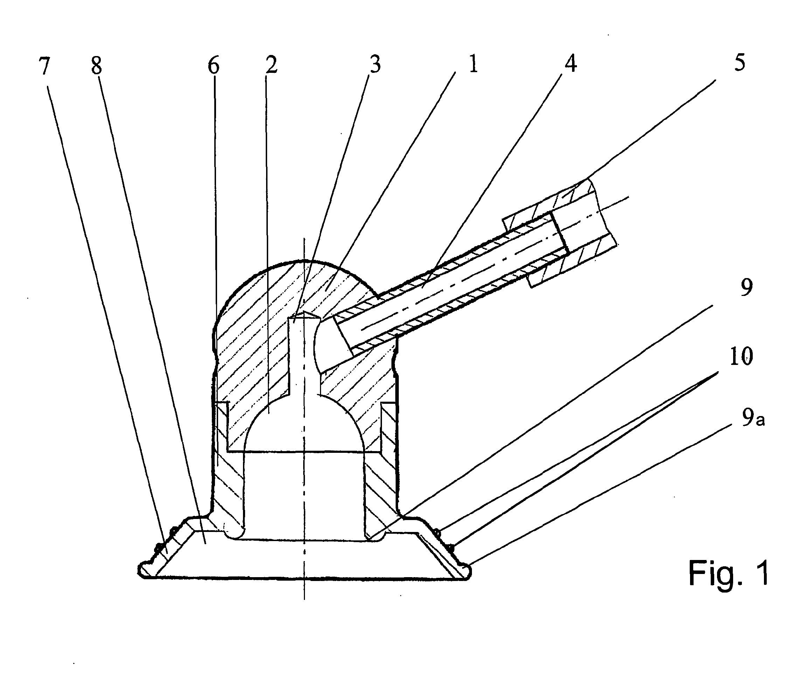

Der in der Figur dargestellte Kopfteil des Doppelstethoskops besteht im Wesentlichen aus einem halbkugelförmigen Schlauchanschlusskörper 1, ein oder zwei Rohrstutzen 4 zur Weiterleitung der Körpergeräusche aus dem Schalltrichter 7 über das Schlauchsystem und die Ohrstücke in das Ohr des Untersuchenden, sowie aus einem einstückigen Doppelschalltrichter.

Der Schlauchanschlusskörper 1 ist aus Edelstahl gefertigt und entspricht damit dem hohen medizinischen Standard. Zur guten und sicheren Handhabung des Bruststückes ist am maximalen Umfang des kugelförmigen Schlauchanschlusskörpers 1 ein umlaufender Einstich in Form eines Kreissegments eingearbeitet, der ebenso wie die kugelförmige Oberfläche leicht zu reinigen und zu desinfizieren ist. Zur Weiterleitung der Körpergeräusche aus dem Schalltrichter 7 weist der Schlauchanschlusskörper 1 einen rotationsellipsoiden Schallraum 2 sowie in dessen Verlängerung eine Schallleitungsbohrung 3 auf, die je nach Kundenwunsch mit einem oder zwei Rohrstutzen 4 akustisch verbunden sind. Die starke Wandung des als Drehteil hergestellten Schlauchanschlusskörpers 1 dämmt hierbei eventuell auftretende Eigengeräusche des Untersuchenden.

Auf einer umlaufenden Außennut an der Öffnung des Schlauchanschlusskörpers 1 ist ein aus einem weichelastischen Kunststoff gegossener Schalltrichter 7 aufgesteckt und in dieser kraftschlüssig gehalten. Hierzu besitzt das hohlzylindrische Anschlussstück 6 des Schalltrichters 7 eine zu der umlaufenden Außennut des Schlauchanschlusskörpers 1 adäquate Innennut. Über einen Einstich in der umlaufenden Außennut des Schlauchanschlusskörpers 1 und einen adäquaten Wulst in der Innennut des hohlzylindrischen Anschlussstückes 6 kann der Schalltrichter 7 zusätzlich formschlüssig in der Fügestelle gesichert werden. Natürlich besteht auch die Möglichkeit, beide Einzelteile mit einem Klebstoff stoffschlüssig zu verbinden.

Zu der Funktionsseite hin erweitert sich das hohlzylindrische Anschlussstück 6 in einen Schalltrichter 7 mit der bekannten geometrischen Form und bildet somit den Schallkollektorraum 8. Einerseits unterhalb des hohlzylindrischen Anschlussstückes 6, in den Schallkollektorraum 8 gerichtet, und andererseits an der Öffnung des Schalltrichters 7, weist der weichelastische Schalltrichter 7 jeweils umlaufende Abdichtwulste 9, 9a auf, die in ihren Querschnitten bei der dargestellten Ausführungsform kreisförmig gestaltet sind.

Bei der allgemeinen Auskultation wird das Bruststück von dem Behandelnden leicht an die entsprechende Körperoberfläche gedrückt, so dass der umlaufende Abdichtwulst 9a an der Öffnung des Schalltrichters 7 diese exakt abdeckt.

Beim interkostalen Aufsetzen des Bruststückes dagegen wird der Schalltrichter 7 unter größerem Druck des Untersuchenden so weit deformiert, dass der unterhalb des hohlzylindrischen Anschlussstückes 6 umlaufende Außenwulst 9 auf der Körperoberfläche zur Anlage kommt. Damit wird ohne Verstellen einer Mechanik ein zweiter Schalltrichter wirksam, dessen Öffnungsquerschnitt kleiner ist als der des äußeren Schalltrichters 7.

Die Verwendung von weichelastischem Kunststoff zur Herstellung des Schalltrichters 7 sowie die kreisförmige Querschnittgestaltung der umlaufenden Abdichtwulste 9, 9a ermöglichen bei unterschiedlichen Untersuchungsbedingungen ein vollständiges Abdecken der zu untersuchenden Körperoberfläche und somit eine exakte Lokalisation zum Patienten. Mit der konstruktiven Ausgestaltung des Kopfteils werden äußere Fremdgeräusche voll ausgeschlossen, aufzunehmende Körpergeräusche dagegen ohne Verluste bis in das Ohr des Untersuchenden übertragen.

Die konvexe oder konkave Gestaltung des Schalltrichters 7 zwischen den umlaufenden Abdichtwulsten 9, 9a und eine oder mehrere umlaufende Stabilisierungswulste 10 verhindern das seitliche Abknicken des Schalltrichters 7 bei eventuell eingeleiteten Querkräften auf das Bruststück durch den Untersuchenden.

Es ist bekannt, dass Stethoskope als Überträger von pathogenen, multiresistenten Hospitalkeimen ganz oben auf der Liste der kontaminierten Gegenstände stehen. Der einfache, kompakte Aufbau des Bruststückes ermöglicht daher eine einfache Reinigung und Desinfektion dieser Baugruppe.

Eine antimikrobielle Ausstattung des weichen Polyurethan-oder Silikon-Schalltrichters 7 wird durch die Zugabe von Nanosilber in die zu verarbeitende Gießmasse erreicht. Hierbei werden vorzugsweise 2 Vol% Nanosilber vor dem Vergießen dem Polyurethan bzw. Silikon beigemischt und mechanisch in diesen homogen verteilt.

Bei derartigem Nanosilber handelt es sich um Nanoteilchen aus Silber, die im Kunststoffmaterial des Schalltrichters möglichst gleichmäßig verteilt eingebracht werden. Die Silberpartikel geben kontinuierlich positiv geladene Ionen ab, wodurch evtl. an der Oberfläche des Schalltrichters anhaftende Bakterien bereits nach kurzer Zeit abgetötet sind. Es ist verständlich, dass die Beifügung von Nanosilber in das Ausgangsmaterial des Schalltrichters unabhängig von der speziellen Formgebung dieses Schalltrichters ist. Die antimikrobielle bzw. antibakterielle Wirkung des Nanosilbers kann daher auch an konstruktiv abgewandelten Schalltrichtern von Stethoskopen zum Einsatz gebracht werden.

### Bezugszeichenliste

- 1: Schlauchanschlusskörper
- 2: rotationsellipsoider Schallraum
- 3: Schallleitungsbohrung
- 4: Rohrstutzen
- 5: Schlauchsystem
- 6: hohlzylindrisches Anschlussstück
- 7: Schalltrichter
- 8: Schallkollektorraum
- 9: erster umlaufender Abdichtwulst
- 9a: zweiter umlaufender Abdichtwulst
- 10: umlaufender Stabilisierungswulst

## Patentansprüche

1. Doppelstethoskop mit einem Schalltrichter (7), der einen Schallkollektorraum (8) umfasst und der über einen Schlauchanschlusskörper (1) zum Übertragen von Geräuschen an ein Schlauchsystem (5) angeschlossen ist, **dadurch gekennzeichnet, dass** der Schalltrichter (7) aus einem weichelastischen Material besteht und über ein hohles Anschlussstück (6) mit dem Schlauchanschlusskörper (1) verbunden ist, und dass an dem zum Schallkollektorraum (8) gerichteten Ende des hohlen Anschlussstücks (6) eine erste umlaufende Abdichtwulst (9) angeordnet ist.

2. Doppelstethoskop nach Anspruch 1, **dadurch gekennzeichnet, dass** an der Öffnung des Schalltrichters (7) eine zweite umlaufende Abdichtwulst (9a) angeordnet ist.

3. Doppelstethoskop nach Anspruch 1 oder 2, **dadurch gekennzeichnet, dass** der Schlauchanschlusskörper (1) halbkugelförmig ausgebildet ist und einen rotationsellipsoiden Schallraum (2) aufweist, der in das Anschlussstück (6) geöffnet ist, welches adäquat zum Schallraum einen hohlzylindrischen Querschnitt besitzt, der in den Schallkollektorraum (8) des Schalltrichters (7) geöffnet ist.

4. Doppelstethoskop nach einem der Ansprüche 1 bis 3, **dadurch gekennzeichnet, dass** das Schlauchsystem (5) über einen Rohrstutzen (4) an den Schlauchanschlusskörper (1) angeschlossen ist, wobei zwischen dem innen liegenden Ende des Rohrstutzens (4) und dem Schallraum (2) eine Schalleitungsbohrung (3) verläuft.

5. Doppelstethoskop nach einem der Ansprüche 1 bis 4, **dadurch gekennzeichnet, dass** die umlaufenden Abdichtwulste (9, 9a) im Querschnitt einen Radius aufweisen.

6. Doppelstethoskop nach einem der Ansprüche 1 bis 5, **dadurch gekennzeichnet, dass** der Schalltrichter (7) zwischen den umlaufenden Abdichtwulsten (9, 9a) kegelförmig, konvex oder konkav ausgebildet ist.

7. Doppelstethoskop nach einem der Ansprüche 1 bis 6, **dadurch gekennzeichnet, dass** zwischen den umlaufenden Abdichtwulsten (9, 9a) auf der Außenfläche des Schalltrichters (7) umlaufende Stabilisierungswulste (10) angeordnet sind.

8. Doppelstethoskop nach einem der Ansprüche 1 bis 7, **dadurch gekennzeichnet, dass** der Schlauchanschlusskörper (1) aus Edelstahl gefertigt ist.

9. Doppelstethoskop nach einem der Ansprüche 1 bis 8, **dadurch gekennzeichnet, dass** der weichelastische Kunststoff des Schalltrichters (7) Polyurethan oder ein Silikon ist.

10. Doppelstethoskop nach einem der Ansprüche 1 bis 9, **dadurch gekennzeichnet, dass** der Schalltrichter (7) einstückig mit dem hohlen Anschlussstück (6) ausgebildet ist und aus einem weichelastischen Kunststoff besteht.

11. Doppelstethoskop nach einem der Ansprüche 1 bis 10, **dadurch gekennzeichnet, dass** dem weichelastischen Material des Schalltrichters (7) Nanosilber zugesetzt ist, insbesondere Anteil von etwa 2 Vol%.
